# EUROPEAN PATENT APPLICATION

(11) **EP 1 435 244 A1**
(43) Date of publication of application: **07.07.2004**
(21) Application number: 02785923.0
(22) Date of filing: 11.10.2002
(51) Int. Cl.: A61K 45/00, A61K 38/05, A61K 38/06, A61P 3/06, A61P 9/10, A61P 43/00, G01N 33/50

(54) **DRUGS AMELIORATING HYPO-HDL CHOLESTEROLEMIA**

(30) Priority: 12.10.2001 JP 2001314756
(71) Applicant: Grelan Pharmaceutical Co., Ltd., Hamura-shi, Tokyo 205-8501 (JP)
(72) Inventor: YOKOYAMA, Shinji, Nagoya-shi, Aichi 467-0024 (JP); ARAKAWA, Reijiro, c/o Grelan Research Center, Hamura-shi, Tokyo 205-8501 (JP)
(74) Representative: von Kreisler, Alek, Dipl.-Chem.
(86) International application number: PCT/JP2002/010620
(87) International publication number: WO 2003/033023

(57) **Abstract**

The present invention is to provide an agent for low HDL-cholesterolemia, a prophylactic and/or therapeutic antiarteriosclerosis agent as well as a method for preventing and treating low HDL-cholesterolemia, arteriosclerosis and their related diseases or disorders, with emphasis given to improvement in HDL, without resorting to genetic engineering technology. Further, the present invention is to provide a clinically effective agent for low HDL-cholesterolemia and a prophylactic and/or therapeutic antiarteriosclerosis agent, comprising at least one cysteine protease inhibitor as an active ingredient, thereby increasing a quantity of expressed ABCA1 and elevating blood HDL levels, without using the genetic engineering technology.

## Description

### FIELD OF THE INVENTION

The present invention relates to a therapeutic agent for low high-density lipoprotein (HDL)-cholesterolemia in which a cysteine protease inhibitor is used as an active ingredient and to a prophylactic and/or therapeutic agent for arteriosclerosis (antiarteriosclerosis agent) comprising said agent for low HDL-cholesterolemia.

### BACKGROUND OF THE INVENTION

HDL (high-density lipoprotein) is mainly synthesized in and secreted from the liver and epithelial cells of the small intestine. Immediately after secretion, HDL is in a form of discoidal particle containing apoprotein A-I (also called apoAI) and phospholipid as its major constituents, and also called nascent HDL. This nascent HDL receives, in blood, free cholesterol from cell membranes of peripheral cells or produced in the hydrolysis course of other lipoproteins, and forms mature spherical HDL while holding, at its hydrophobic center, cholesterol ester converted from said cholesterol by the action of LCAT (lecithin cholesterol acyltransferase).

HDL plays an extremely important role in physiological function in terms of lipid metabolism called "reverse cholesterol transport system" which takes, in blood, excessive cholesterol out of peripheral tissues and transports it to the liver. The reverse cholesterol transport system is considered to cause a prophylactic action on arteriosclerosis.

In addition to the above biochemical findings, many epidemiological studies have revealed a relationship between blood HDL cholesterol levels and arteriosclerosis, namely, lower HDL cholesterol levels resulting in a higher incidence of arteriosclerosis. Thus, it is extremely important to relieve low HDL-cholesterolemia in view of prevention and treatment of arteriosclerosis.

At present, blood levels of HDL can be determined by referring to the cholesterol levels of HDL fraction (hereinafter referred to as HDL cholesterol) that can be easily measured clinically. As for Japanese, a person bearing the blood HDL cholesterol level of less than 40 mg/dl has been generally diagnosed with "low HDL-cholesterolemia".

Low HDL-cholesterolemia is found at a high incidence in various disorders such as hyperlipidemia, cerebral infarction, obesity and diabetes mellitus, in addition to arteriosclerosis. This disorder is also found in various genetic diseases including Tangier disease.

It was often difficult for patients with inherited diseases to receive treatment for low HDL-cholesterolemia. Further, in treating low HDL-cholesterolemia in association with disorders such as arteriosclerosis, hyperlipidemia, cerebral infarction, obesity and diabetes mellitus, an effective agent for low HDL-cholesterolemia directly targeting the improvement of the HDL level has not been made available and its arrival has been desired.

Further, an interesting finding has been obtained regarding HDL containing apolipoprotein as a major constituent, different from the aforementioned HDL which is synthesized in and secreted from the liver and epithelial cells of the small intestine.

More particularly, HDL synthesized in and secreted from astrocytes in the brain contains apolipoprotein E (also called apoE) and phospholipid as major constituents. It has been found that apoE is involved in repairing a damaged central nervous system, and that repairing action on the damaged nervous system of brain astrocytes in the presence of the apoE delays progression of nervous disorders such as Altzheimer's disease (Schiefermeier et al., Stroke, 31(9), 2068-2073(2000)). Therefore, an increase in apoE-HDL synthesized in and secreted from brain astrocytes is extremely important in preventing and treating nervous disorders or diseases involved in a damaged central nervous system.

In recent years, Bodzioch et al. (Nat. Genet., 22, 347-351(1999)), Brooks-Wilson et al. (Nat. Genet., 22, 336-345(1999)) and Rust et al. (Nat. Genet., 22, 347-351(1999)) have identified ABCA1 (ATP-binding cassette transporter 1) as a causative gene of Tangier disease which exhibits extreme low levels of HDL, resulting in an increased focus on the relationship between low HDL-cholesterolemia and the ABCA1 gene.

ABCA1 is a protein mainly present in cell membranes of various organs such as the liver, small intestine, placenta and adrenal gland, and considered to be involved in membrane transport of various substances such as amino acids, vitamins and saccharides (Annu. Rev. Cell, Biol., 8, 67-113(1992)).

According to a recent finding regarding the above matter, it was reported that since patients with Tangier disease underwent mutations of the ABCA1 gene, they were deficient in expressing ABCA1 necessary for lipid metabolism, thereby leading to disorders for a flux of cholesterol into the extracellular pathway and the disappearance of HDL.

In addition to the above finding, Richard et al. (J. Clin. Invest., 104, R25-R31(1999)) and Von Eckardstein et al. (The FASEB J., 15, 1555-1561(2001)) found that incorporation of ABCA1 gene accelerated the formation of HDL. Several trials are now in progress where genetic engineering technology is used to increase the quantity of in vivo expressed ABCA1, thus elevating or regulating the levels of HDL cholesterol.

For example, in WO 00/78971 and WO 00/78972, such techniques are disclosed where the ABCA1 polypeptide is directly introduced into a host cell to elevate the expression and activity of ABCA1, thereby increasing the efflux of cholesterol and levels of HDL. Further, in WO 01/15676, such technology is disclosed where a certain substance is used to facilitate the transcription and translation of the ABCA1 gene to increase the expression and activity of ABCA1, thereby controlling the levels of HDL cholesterol and triglyceride.

As explained above, it is extremely important to ameliorate low HDL-cholesterolemia in terms of prevention and treatment of arteriosclerosis. It is greatly desired to develop an effective agent for low HDL-cholesterolemia which targets a direct improvement of the HDL level. On the basis of the recently-obtained findings on Tangier disease, trials that increase the expression of ABGA1 to raise or control the level of HDL cholesterol have been conducted. However, all of the agents employed in these trials have been based on the genetic engineering technology. Thus, even if these agents are effective in treating genetic disease, they cannot effectively remedy low HDL-cholesterolemia found in patients with diseases such as arteriosclerosis, hyperlipidemia, cerebral infarction, obesity and diabetes mellitus, who are significantly larger in the number than those afflicted with said genetic disease.

Under these circumstances, there is still a greatly desirable need to provide such an agent for low HDL-cholesterolemia that can effectively target improving the HDL level while it does not require genetic engineering technology.

### SUMMARY OF THE INVENTION

In order to find an effective agent for low HDL-cholesterolemia free of using genetic engineering technology and capable of targeting an increase in HDL levels, the present inventors have employed an assay system whose index is an elevated expression of ABCA1 in order to examine a variety of substances. As a result, the present inventors have succeeded in finding that cysteine protease inhibitor is capable of elevating the expression of ABCA1 to a great extent in a sustained manner, and accomplished the present invention.

The present invention provides the following:
1) a therapeutic agent for low HDL-cholesterolemia which comprises at least one of cysteine protease inhibitors as an active ingredient;
2) the therapeutic agent for low HDL-cholesterolemia according to Claim 1 wherein the cysteine protease inhibitor is derived from natural or non-natural sources;
3) the therapeutic agent for low HDL-cholesterolemia according to the above 2) wherein the naturally occurring cysteine protease inhibitor is derived from microorganisms, plants or animals;
4) the therapeutic agent for low HDL-cholesterolemia according to the above 3) wherein the cysteine protease inhibitor derived from microbial, plant or animal sources is selected from a group consisting of leupeptin, antipain and E-64;
5) the therapeutic agent for low HDL-cholesterolemia according to the above 2) wherein the non-naturally occurring cysteine protease inhibitor is not derived from microbial, plant or animal sources, but synthesized;
6) the therapeutic agent for low HDL-cholesterolemia according to the above 5) wherein the non-naturally occurring cysteine protease inhibitor is ALLN or N-acetyl leu-leu-methioninal;
7) a prophylactic or therapeutic agent for arteriosclerosis which comprises at least one of the agents for low HDL-cholesterolemia according to any of the above 1) to 6), and
8) a screening method for a substance capable of accelerating the formation of HDL which comprises use of THP-1 cells for providing the expression of ABCA1 as an index.

In another aspect, the invention provides the following:
9) a method for the prevention and/or treatment of low HDL-cholesterolemia and/or arteriosclerosis and/or their related diseases and disorders which comprising administering an effective amount of at least one member of cysteine protease inhibitors to an animal with low HDL-cholesterolemia or arteriosclerosis risk factors and;
10) use of cysteine protease inhibitor for manufacturing an agent for low HDL-cholesterolemia or prophylactically and/or therapeutically arteriosclerosis comprising an effective amount of said cysteine protease inhibitor in admixture with a pharmaceutically acceptable carrier.

The above objectives and other objectives, features, advantages, and aspects of the present invention are readily apparent to those skilled in the art from the following disclosures. It should be understood, however, that the description of the specification including the following best modes of carrying out the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

### BRIEF DESCRIPTION OF THE DRAWINGS

FIG 1 is a graph showing results obtained in Assay Example 1.
FIG 2 is a graph showing results obtained in Assay Example 2.
FIG 3 is a graph showing results obtained in Assay Example 3.

### BEST MODES OF CARRYING OUT THE INVENTION

The present invention provides an agent for low HDL-cholesterolemia wherein the cysteine protease inhibitor is used as an active ingredient, a prophylactic and/or therapeutic arteriosclerosis agent based on said agent for low HDL-cholesterolemia and a method for preventing and treating low HDL-cholesterolemia and/or arteriosclerosis as well as their related diseases and disorders.

The term "cysteine protease inhibitor" as used herein refers to a substance exerting an inhibitory action on cysteine protease (protease having an SH group as its active center, "Seikagaku Jiten (Dictionary of Biochemistry)", 3^{rd} Edition, published by Tokyo Kagaku Dojin Co., Ltd., 637 (1998)) and may be derived from natural sources or non-natural sources.

The aforementioned naturally occurring cysteine protease inhibitor includes those derived from microbial, plant or animal sources, which refer to substances produced and isolated from microorganisms, plants or animal tissues. The aforementioned microorganism-, plant- or animal-derived cysteine protease inhibitor includes leupeptin ("Seikagaku Jiten (Dictionary of Biochemistry)", 3^{rd} Edition, published by Tokyo Kagaku Dojin Co., Ltd., 1531 (1998)), antipain ("Seikagaku Jiten (Dictionary of Biochemistry)", 3^{rd} Edition, published by Tokyo Kagaku Dojin Co., Ltd., 110 (1998)), E-64 (L-trans-epoxysuccinyl-leucylamido-(4-guanidino)butane; "Seikagaku Jiten (Dictionary of Biochemistry)", 3^{rd} Edition, published by Tokyo Kagaku Dojin Co., Ltd., 150 (1998)), etc. The cysteine protease inhibitor also includes microorganism-derived cysteine protease inhibitors disclosed in Published Japanese Unexamined Patent Application No. H1D-77276 (JP, A, H10-77276 (1998)); microorganism-derived cysteine protease inhibitors disclosed in JP, A, H7-48340 (1995); etc. Among these inhibitors, leupeptin, antipain and E-64 are preferable.

The aforementioned non-naturally occurring cysteine protease inhibitors are synthetic substances which are not derived from microorganism, plant or animal sources. The above non-naturally occurring cysteine protease inhibitors include, for example, ALLN (N-acetyl-leu-leunorleucinal; J. Enzyme Inhibition, 3, 195(1990)), N-acetyl leu-leu-methioninal (J. Enzyme Inhibition, 3, 195(1990)), etc. The non-naturally occurring cysteine protease inhibitors also include cysteine protease inhibitors disclosed in the following documents and patent gazettes as well as non-naturally occurring cysteine protease inhibitors cited in the following documents and patent gazettes. Among these inhibitors, ALLN and N-acetyl leu-leu-methioninal are preferable.

Biochemical Biophysical Research Communications, 157, 1117(1988); J. Enzyme Inhibition, 3, 195(1990); JP, A, H7-48340 (1995); JP, A, H8-151355 (1996); JP, A, HB-325282 (1996); JP, A, H9-165360 (1997); JP, A, H9-169752 (1997); JP, A, H9-295996 (1997); JP, A, H10-147564 (1998); JP, A, H10-147566 (1998); JP, A, H11-1491 (1999); JP, A, H11-171881 (1999); JP, A, 2001-139534; JP, A, 2001-233847; Japanese Translation of PCT International Application (Kohyo) No. H11-507912 (JP, A, H11-507912 (1999)); JP, A, 2000-510447; JP, A, 2001-505889; JP, A, 2001-506596; JP, A, 2001-506614; WO96/25408; WO97/03060; WO98/47887; WO98/01133 and WO98/04539.

The aforementioned cysteine protease inhibitors are all known substances. The inhibitors isolated and synthesized according to the methods disclosed in the above documents and patent gazettes and other techniques or commercially available inhibitors may be used in the present invention, but the preferable ones are at the grade that can be appreciated as a pharmaceutical drug in terms of quality and stability.

The cysteine protease inhibitor includes, for example, peptidylaldehyde derivatives that may have chemical structures as disclosed in Biochemical Biophysical Research Communications, 157, 1117(1988), J. Enzyme Inhibition, 3, 195 (1990), etc., including leupeptin, antipain, ALLN, N-acetyl leu-leu-methioninal and others; for example, sulfonamidated peptidylaldehyde derivatives that may have chemical structures as shown in JP, A, H7-48340 (1995), JP, A, H10-147564 (1998), JP, A, H10-147566 (1998), JP, A, 2001-233847, etc.; for example, E-64, epoxy succinic acid amide derivatives that may have chemical structures as shown in JP, A, H8-325282 (1996), JP, A, H10-77276 (1998), JP, A, H10-147566 (1998), WO97/03060, WO98/47887, etc.; for example, oxygen-containing heterocyclic ring derivatives having at least one peptide linkage that may have chemical structures as shown in JP, A, H9-169752 (1997), JP, A, H11-171881 (1999), WO96/25408, W098/04539, etc.; peptides that may have chemical structures as shown in JP, A, H9-165360 (1997), JP, A, H11-1491 (1999), JP, A, H9-295996 (1997), etc.; for example, acylamino aldehyde derivatives that may have chemical structures as shown in JP, A, H8-151355 (1996), etc.; for example, valine derivatives that may have chemical structures as shown in JP, A, 2001-139534, etc.; for example, α-(1,3-dicarbonyl enol ether)methylketone derivatives that may have chemical structures as shown in JP, A, H11-507912 (1999), etc.; for example, 4-oxa-1-azabicyclo[3,2,0]heptane derivatives that may have chemical structures as shown in JP, A, 2000-510447, etc.; for example, benzamide aldehyde derivatives that may have chemical structures as shown in JP, A, 2001-505889, JP, A, 2001-506596, etc.; for example, keto benzamide derivatives that may have chemical structures as shown in JP, A, 2001-506614, etc.; for example, proline derivatives that may have chemical structures as shown in WO98/01133, etc; other inhibitor compounds; and their variants and modified compounds.

The aforementioned agent for low HDL-cholesterolemia is a drug which increases the blood level of HDL cholesterol in the low HDL-cholesterolemia based on clinically diagnostic standards, thereby ameliorating low HDL-cholesterolemia. Typically, in Japanese, a person bearing the blood HDL cholesterol level of less than 40 mg/dl is diagnosed as low HDL-cholesterolemia. When such clinical standards are revised in the future, low HDL-cholesterolemia based on the revised standards will be used in the present invention.

Low HDL-cholesterolemia is found in genetic disorders such as Tangier disease and also in diseases such as arteriosclerosis, hyperlipidemia, cerebral infarction, obesity, diabetes mellitus, abnormal thyroid function, hepatic cirrhosis, myeloma, chronic renal failure, chronic inflammatory bowel diseases (such as Crohn's disease and ulcerative colitis) and others. The agent for low HDL-cholesterolemia of the present invention may be used in any low HDL-cholesterolemia associated with the above diseases and disorders excluding inherited disorders such as some Tangier disease in which ABCA1 is not normally synthesized in vivo.

In addition, the agent for low HDL-cholesterolemia of the present invention is excellently active in accelerating the formation of HDL as will be apparent from examples disclosed herein below and is thus capable of increasing blood HDL levels. As aforementioned, the blood HDL plays an important role in the reverse cholesterol transport system which is considered to act prophylactically on arteriosclerosis. Accordingly, the agent for low HDL-cholesterolemia of the present invention capable of increasing blood HDL levels is effective in both preventing and treating arteriosclerosis.

In low HDL-cholesterolemia, a decreased level of HDL results in a poor function of the reverse cholesterol transport system, thus rendering it difficult to transfer cholesterol accumulated on the vascular wall into the extravascular pathway, whereby arteriosclerosis will be aggravated. The agent for low HDL-cholesterolemia of the present invention is capable of improving such conditions and employed not only prophylactically but also therapeutically against arteriosclerosis.

Further since the agent for low HDL-cholesterolemia of the present invention is active in elevating the level of apoE-HDL in the brain as well, thereby accelerating repair of damaged central nervous system, it is also pharmaceutically effective in both preventing and treating diseases and disorders related to a damaged central nervous system (such as Alzheimer's disease).

The present invention also provides a screening method for a substance capable of accelerating the formation of HDL which comprises use of THP-1 cells (human leukemia cells) to obtain the expression of ABCA1 as an index.

More particularly, a substance to be screened is added to macrophage obtained through cultivation and differentiation of the THP-1 cell to determine a quantity of intracellularly expressed ABCA1, thereby allowing us to discover a substance accelerating the formation of HDL. Substances to be screened are not particularly limited to but may include those derived from either natural or non-natural sources. Further, a quantity of expressed ABCA1 may be determined by any genetic engineering method in general. For example, the method described in Assay Example 1 or 2 may be used in the present invention.

The agent for low HDL-cholesterolemia and the prophylactic and/or therapeutic agent for arteriosclerosis of the present invention may be administered independently or preferably in a pharmaceutical form to which pharmacologically acceptable additives (or carriers) are added. They are administered via oral or injection routes. Ingredients selected from known pharmaceutical additives (hereinafter referred to as pharmaceutical ingredients) may be appropriately used in the aforementioned pharmaceutical preparations or formulations for either of such administration routes. Specific known pharmaceutical additives may be suitably selected from those listed in (1) Iyakuhin Tenkabutsu Handbook (Handbook of Pharmaceutical Excipients), Maruzen Co., Ltd., (1989), (2) Iyakuhin Tenkabutsu Jiten (Dictionary of Pharmaceutical Additives), 1^{st} Edition, The Yakuji Nippo Limited (1994), (3) Iyakuhin Tenkabutsu Jiten Tsuiho (Dictionary of Pharmaceutical Additives, Suppl.), 1^{st} Edition, The Yakuji Nippo Limited (1995) and (4) Yakuzai Gaku (Pharmacology), Revised 5^{th} Edition, Nankodo Co., Ltd. (1997), depending on formulation applications and administration routes.

For example, for oral administration, the above additives include any pharmaceutical ingredients that can form an oral drug and accomplish the purpose of the present invention. However, an oral drug is formed by selecting known pharmaceutical ingredients such as vehicles or excipients, binders, disintegrants, lubricants or glidants and coating agents. Specific oral drugs include tablets, capsules, granules, microgranules, powders, syrups, etc. Further, said oral drugs include pharmaceutically controlled delivery preparations (e.g., rapid release drugs and sustained release drugs) in which known pharmaceutical ingredients are employed to control in vivo release of the cysteine protease inhibitor, an active ingredient.

For injection, the above additives include pharmaceutical ingredients which can form either aqueous injections or non-aqueous injections. Typically, the additives are known pharmaceutical ingredients such as resolvents, solution adjuvants, suspending agents, buffers, stabilizing agents and preservatives. Further, the additives may include known pharmaceutical ingredients constituting injectable powders which can be dissolved or suspended upon administration. Pharmaceutical ingredients for aqueous injections include distilled water for injection, sterilized isotonic salt solutions (including mono-sodium or di-sodium phosphate, sodium chloride, potassium chloride, calcium chloride, magnesium chloride, etc. or mixtures of these salts).

An effective dose of the agent for low HDL-cholesterolemia and the prophylactic and/or therapeutic agent for arteriosclerosis of the present invention will be selected depending on age, bodyweight, conditions of low HDL-cholesterolemia and arteriosclerosis, and absence or presence of complications in patients who receive such agents. Such effective dose will be adjusted appropriately and typically ranges from about 1 mg to 3,000 mg/day for oral administration and from about 0.1 mg to 1,000 mg/day for injection.

In view of the foregoing disclosures, it will be understood that the invention provides a method for preventing and/or treating low HDL-cholesterolemia and/or arteriosclerosis and/or their related diseases or disorders which includes administering an effective dose of at least one of cysteine protease inhibitors to living body with low HDL-cholesterolemia or arteriosclerosis risk factors, as well as use of cysteine protease inhibitor for manufacturing an agent for low HDL-cholesterolemia or prophylactically and/or therapeutically arteriosclerosis including an effective dose of said cysteine protease inhibitor in admixture with a pharmaceutically acceptable carrier. Specific modes of the method and application can be implemented according to the above description.

### Examples, etc.

Described below are examples, including assay examples and formulation examples of the present invention, which are provided only for illustrative purposes, and not to limit the scope of the present invention. All the examples were carried out or can be carried out, unless otherwise disclosed herein specifically, by standard techniques which are well known and conventional to those skilled in the art.

### Assay Example 1

### Action of ALLN on increase of the expression of ABCA1

### <Test method>

THP-1 cells (human leukemia cell, American Type Culture Collection) were cultured for 72 hours in a 10%FBS-RPMI1640 medium (Iwaki Glass Co., Ltd.), together with PMA (phorbol myristate acetate, Wako Pure Chemical Industries Ltd.) to obtain differentiated macrophages. The medium was replaced with 0.2%BSA-RPMI 1640 (Iwaki Glass Co., Ltd.) to which ALLN (50µM, Sigma) was added, and examined for the time-course of intracellular ABCA1 expression according to the method illustrated herein below.

ALLN-treated cells and control cells cultivated in ALLN-free media were hypotonically disrupted in 5mM Tris-HCl (pH8.5), and then centrifuged (650g, for 5 minutes) to precipitate nuclear fractions. The supernatant was centrifuged at 105,000g for 30 minutes to collect a total membrane fraction. The total membrane was dissolved in an aqueous solution containing 0.9M urea, 0.2% triton X-100 (Wako Pure Chemical Industries Ltd.), and 0.1% dithiothreitol (Wako Pure Chemical Industries Ltd.), followed by addition of 10% lithium dodecyl sulfate solution (Wako Pure Chemical Industries Ltd.) at a volume ratio of 1/4. The resultant was subjected to electrophoretic separation using 10% SDS-7% polyacrylamide gel (Wako Pure Chemical Industries Ltd.). The separated protein was fixed and transferred onto PVDF membrane (Bio-Rad). Each sample was then subjected to immunoblotting with rabbit anti-human ABCA1 antibody (self-made purification by an ordinary method) to quantitate expressed ABCA1 proteins. The obtained bands were read for density and size with Scion Image (image analysis software, Scion) to digitize the results.

### <Results>

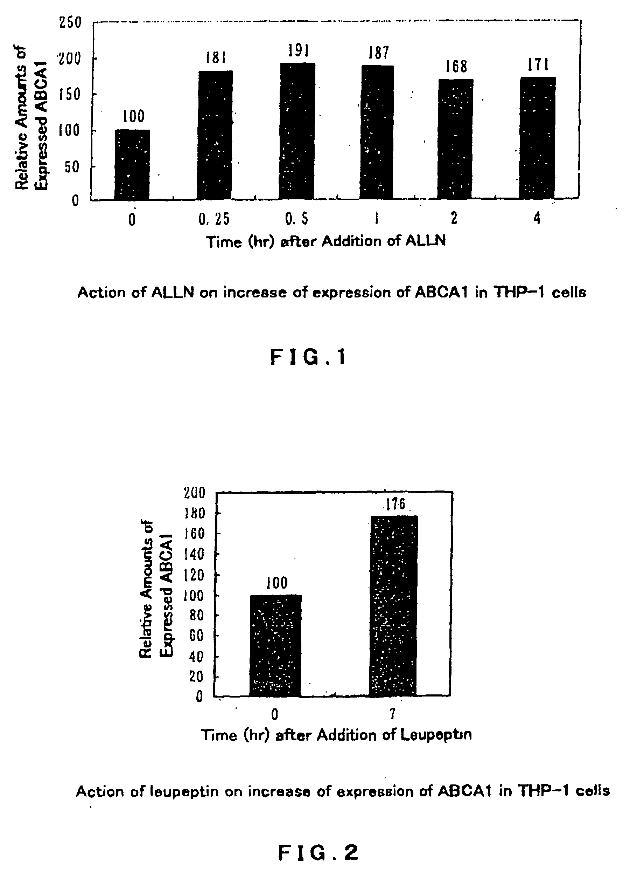

It was found that THP-1 cells cultured in ALLN-added medium exhibited a remarkable intracellular expression of ABCA1, as compared with the control cells. As shown in FIG 1, the action of increasing the expression of ABCA1 was found until 4 hours after addition of ALLN, and, more particularly, the action was found 15 minutes after addition of ALLN. When the expression of ABCA1 was designated as 100 on the basis of the control cells (0 time after addition of ALLN), the intensity profile of the action was 168 to 191. Further, the action of increasing the expression of ABCA1 was found even 24 hours after addition of ALLN.

### Assay Example 2

### Action of leupeptin on increase of the expression of ABCA1

In the same manner as in Assay Example 1, THP-1 cells were cultured in a medium to which leupeptin was added in place of ALLN, and compared with control cells non-treated with leupeptin. As a result, leupeptin-treated cells exhibited a remarkable increase in the intracellular expression of ABCA1, as compared with the control cells. FIG 2 shows relative amounts of expressed ABCA1 in the leupeptin-treated cells (7 hours after addition of leupeptin) to the control cells (0 time after addition of leupeptin). The leupeptin-induced action of elevating the expression of ABCA1 was found even 24 hours after addition of leupeptin to the medium.

### Assay Example 3

### Action of ALLN on acceleration of the formation of HDL

### <Test method>

In the same manner as in Arakawa et al., J. Lipid Res., 41, 1952-1962(2000), THP-1 cells were used for quantitative analysis of cholesterols and phospholipids transported into the medium on the basis of apoAI-dependent HDL formation. ALLN-added cells were compared with those free of ALLN addition.

### <Results>

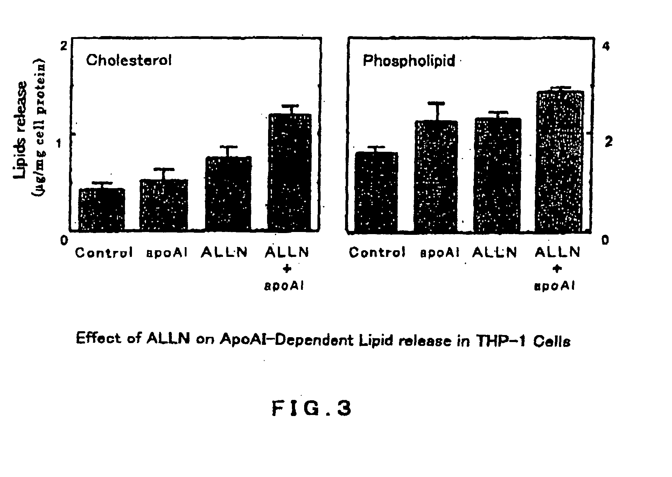

It was found that amounts of apoAI-dependently transported cholesterols and phospholipids increase (i.e., the formation of HDL was accelerated) in both apoAI & ALLN-added cells, as compared with apoAI & ALLN-free cells (control group) and cells to which apoAI alone was added (apoAI-added group). The results are shown in FIG 3.

As explained herein above, it has been verified from these assays that the agent for low HDL-cholesterolemia of the present invention exhibited an action of increasing the expression of ABCA1 without resorting to the genetic engineering technology, thereby accelerating the formation of HDL.

The action of accelerating the expression of ABCA1 by the cysteine protease inhibitor according to the present invention was found immediately after addition of the cysteine protease inhibitor to the cell medium, which was indicated not due to increased intracellular synthesis of ABCA1 as obtained by the conventional genetic engineering technology, but due to an utterly new mechanism, i.e., inhibited metabolism of ABCA1.

### Formulation Example 1

| | |
|---|---|
| Cysteine protease inhibitor according to the present invention | 100 mg |
| Lactose | 100 mg |
| Corn starch | 28 mg |
| Magnesium stearate | 2 mg |

The ingredients were formulated into capsules according to known methods specified in The Japanese Pharmacopoeia, 13^{th} Edition (JP XIII), General Rules for Preparations.

### Formulation Example 2

The cysteine protease inhibitor (50 mg) of the present invention was dissolved in an aqueous isotonic solution prepared from 10mL of distilled water for injection containing an appropriate amount of sodium chloride. The resulting mixture was dispensed to each ampule, and then subjected to sterilization after sealing to obtain injections.

### INDUSTRIAL APPLICABILITY

As explained above, the agent of the present invention comprising at least one cysteine protease inhibitor as an active ingredient can increase the quantity of ABCA1 expressed by a mechanism different from the prior art, without resorting to the above conventional genetic engineering techniques, thereby leading to an increase in the blood HDL level. Therefore, the agent serves effectively as an agent for relieving low HDL-cholesterolemia frequently found in various diseases such as arteriosclerosis. Further, the agent is effective in preventing and treating arteriosclerosis due to the action of ameliorating low HDL-cholesterolemia. It is also possible to prevent and/or treat diseases and disorders by administering the cysteine protease inhibitor of the present invention to animals with risk factors of low HDL-cholesterolemia and arteriosclerosis.

While the present invention has been described specifically in detail with reference to certain embodiments and examples thereof, it would be apparent that it is possible to practice it in other forms. In light of the disclosure, it will be understood that various modifications and variations are within the spirit and scope of the appended claims.

## Claims

1. A therapeutic agent for low HDL-cholesterolemia which comprises at least one of cysteine protease inhibitors as an active ingredient.

2. The therapeutic agent for low HDL-cholesterolemia according to Claim 1 wherein the cysteine protease inhibitor is derived from natural or non-natural sources.

3. The therapeutic agent for low HDL-cholesterolemia according to Claim 2 wherein the naturally occurring cysteine protease inhibitor is derived from microbial, plant or animal sources.

4. The therapeutic agent for low HDL-cholesterolemia according to Claim 3 wherein the cysteine protease inhibitor derived from microbial, plant or animal sources is selected from the group consisting of leupeptin, antipain and E-64.

5. The therapeutic agent for low HDL-cholesterolemia according to Claim 2 wherein the non-naturally occurring cysteine protease inhibitor is not derived from microbial, plant or animal sources but synthetic.

6. The therapeutic agent for low HDL-cholesterolemia according to Claim 5 wherein the non-naturally occurring cysteine protease inhibitor is ALLN or N-acetyl leu-leu-methioninal.

7. A therapeutic or prophylactic agent for arteriosclerosis which comprises at least one of said therapeutically agents for low HDL-cholesterolemia according to any of Claims 1 to 6.

8. A screening method for a substance capable of accelerating the formation of HDL which comprises use of THP-1 cells for providing the expression of ABCA1 as an index.
